Europäisches Patentamt

European Patent Office

Office européen des brevets

(11) Numéro de publication: **0 325 066**

**A1**

(12) **DEMANDE DE BREVET EUROPEEN**

(21) Numéro de dépôt: 88403181.6

(22) Date de dépôt: 14.12.88

(51) Int. Cl.4: **C12N 15/00** , **A01H 1/00**

(30) Priorité: 14.12.87 FR 8717434

(43) Date de publication de la demande:
26.07.89 Bulletin 89/30

(84) Etats contractants désignés:
AT BE CH DE ES FR GB GR IT LI LU NL SE

(71) Demandeur: **INSTITUT NATIONAL DE LA RECHERCHE AGRONOMIQUE (INRA)**
**145, rue de l'Université**
**F-75341 Paris Cédéx 07(FR)**

(72) Inventeur: **Tepfer, Mark**
**80, rue du Cherche Midi**
**F-75006 Paris(FR)**
Inventeur: **Jacquemont, Mireille**
**Le Clos des Avaux**
**F-84510 Caumont sur Durance(FR)**
Inventeur: **Laucquin, Guy**
**24, rue Joliot Curie**
**F-13470 Carnoux en Provence(FR)**

(74) Mandataire: **Warcoin, Jacques et al**
**Cabinet Régimbeau 26, avenue Kléber**
**F-75116 Paris(FR)**

(54) **Procédé d'obtention de plantes transgeniques, vecteur d'intégration utilisé et plantes obtenues.**

(57) La présente invention concerne un procédé d'obtention de plantes transgéniques en vue de les rendre tolérantes de façon durable aux phytovirus par intégration dans leur génome d'une information génétique codant pour l'expression de molécules d'acide nucléique satellite caractérisé en ce que la séquence d'ADN codant pour l'expression de l'acide nucléique satellite est introduite dans le génome sous la forme d'une seule unité monomérique dont les extrêmités possèdent éventuellement des séquences étrangères.

EP 0 325 066 A1

# PROCEDE D'OBTENTION DE PLANTES TRANSGENIQUES, VECTEUR D'INTEGRATION UTILISE ET PLANTES OBTENUES

La présente invention concerne la lutte contre certains virus et notamment le virus de la mosaique du concombre ou VMC.

Le VMC est un virus à ARN monocaténaire à nucléocapside icosaédrique et non enveloppé. Ce virus est connu pour infecter plus de huit cent espèces végétales représentant pratiquement toutes les familles botaniques (DOUINE et al., 1979).

Les problèmes, notamment économiques, engendrés par ces infestations n'ont pu être résolus à l'heure actuelle ; dans plus de 95 % des cas, en effet, il n'existe aucun moyen de lutte efficace contre le VMC.

En premier lieu, il existe très peu de plantes qui résistent naturellement à ce virus et qui permettraient par croisement, l'introduction d'une résistance efficace dans la majorité des plantes cultivées. Par conséquent, aucun moyen de lutte génétique n'est, à priori, envisageable.

En second lieu, les pucerons transmettent la maladie à grande échelle, selon un mode de transmission non persistant : par simple piqûre sur une plante malade, le VMC est prélevé puis peut être retransmis directement à une autre plante, sans latence chez l'insecte.

Ces infestations se produisent, non seulement à grande échelle, mais aussi chez de nombreuses plantes, qu'elles soient sauvages ou cultivées et pendant toutes les saisons de l'année. Par conséquent, les procédés de désherbage local ou d'élimination de plantes atteintes sont dérisoires et ne pourraient conduire à une éradication de la maladie que s'ils étaient accompagnés d'un anéantissement total des pucerons. Ce qui est impossible. Le VMC a un génome divisé constitué de trois espèces d'ARN génomique plus un ARN sous-génomique. Certaines souches de virus contiennent un composant ARN surnuméraire - l'ARN satellite - qui se comporte comme un réel parasite du virus (DIAZ - RUIZ and KAPER, 1977 ; LOT et al., 1977 ; GOULD et al., 1978). Sa présence se traduit par une forte diminution de la teneur du virus et, par voie de conséquence, de la gravité des symptômes développés par l'hôte infecté.

Un moyen de lutte efficace contre le VMC consisterait à utiliser certaines molécules d'ARN satellite.

Certains auteurs ont tenté d'infecter la plante simultanément par le virus pathogène et par l'ARN satellite dans le but de créer une tolérance audit virus. Une telle tentative est efficace mais très dangereuse du point de vue écologique, compte tenu des grandes capacités de mutation des souches virales. Le danger résulte de la dissémination des souches virales éventuellement mutées, indépendamment des molécules d'ARN satellites. On en arrive donc à une propagation provoquée de la maladie. Cette méthode n'est donc pas applicable à grande échelle.

D'autres auteurs ont proposé la création de plantes transgéniques, à savoir des plantes dont le génome comporte non seulement l'information nécessaire à l'expression du virus mais aussi celle permettant l'expression d'un facteur limitant la synthèse dudit virus. C'est ainsi que POWELL ABEL et al. ont réussi à introduire un gène codant pour la protéine d'enveloppe du virus de la mosaique de tabac (VMT) dans le génome de plantes sensibles au VMT. Cette manipulation génétique a permis de créer un retard du développement de la maladie chez les plantes transgéniques. La résistance au VMC a aussi été obtenue au moyen d'un vecteur comportant l'ADN codant pour la protéine d'enveloppe du VMC par FURASAWA et al (EP 279 433). D'autres plantes ont été rendues tolérantes au virus de la mosaique de la luzerne par introduction d'un gène codant pour la protéine capsidique virale (VAN DUN et al., 1987 ; LOESCHFRIES et al., 1987 ; TUMER et al., 1987).

D'autres types de gènes codant pour l'ARN anti-sens (CUOZZO et al., 1988 ; HEMENWAY et al., 1988), ou dont le transcript correspond à un précurseur de l'ARN satellite viral (GERLACH et al., 1987 ; HARRISON et al., 1987), ont été développés pour conférer la tolérance aux virus chez les plantes. Ces approches diffèrent beaucoup aussi bien en terme de mécanisme de résistance, ou de tolérance, impliquée qu'en terme d'expression de ladite résistance : les gènes codant pour la protéine d'enveloppe ou l'ARN anti-sens confèrent une résistance dont l'effectivité dépend du niveau d'expression du gène introduit et de la concentration de l'inoculum viral ; dans de nombreux cas, ce type de résistance est dépassé par des taux importants d'inoculum. Sauf dans le cas de la résistance conférée par la capside du virus de la pomme de terre X (HEMENWAY et al., 1988), la résistance due aux protéines d'enveloppe peut aussi être vaincue par un inoculum d'ARN viral purifié.

Au contraire, lorsque les gènes de résistance basés sur l'ARN satellite sont utilisés conformément à la présente invention, la résistance est observée quelle que soit la souche de virus, la forme (ARN ou particules virales), la concentration de l'inoculum, et le niveau de transcription du gène de l'ARN satellite.

D'autres laboratoires enfin ont créé des plantes transgéniques exprimant des gènes codant pour des ARN satellites. Cette stratégie a été adoptée par GERLACH et al. (1987) pour le virus des tâches annulaires

EP 0 325 066 A1

du tabac, et, pour le VMC, par C. BAULCOMBE et al. (1986).

Ces auteurs ont, cependant, effectué des manipulations très complexes pour pouvoir intégrer un tel génome dans la mesure où ils se sont basés sur des hypothèses prédisant qu'un ARN transcrit comportant la forme monomérique de l'ARN satellite ne serait pas répliquée efficacement par le virus (EP-O 242 016).

Ces hypothèses sont basées sur la faible infectivité des formes monomériques des cADN de viroïdes ou ARN satellites dont on sait par ailleurs qu'ils se répliquent via des formes multimériques.

C'est pourquoi, ces auteurs ont construit des gènes codant pour des formes oligomérique de l'ARN : 3 copies pour GERLACH et al. (1987) dans le cas du virus du ringspot du tabac et 1,3 ou 2,3 copies ou unités d'ARN satellite pour HARISON et al. (1987) ou BAULCOMBE et al. (1986) (ces derniers ont ainsi obtenu une protection partielle des plantes de tabac contre l'infection par le VMC.).

Or, les constructions de formes oligomériques relèvent d'une démarche complexe et longue. Une telle stratégie ne peut donc pas être envisagée pour exprimer un grand nombre de molécules différentes. Pourtant, cette situation se présentera nécessairement pour créer des variants de satellites désarmés dont la dissémination entre plantes ne sera plus possible et qui ne pourront pas, par simple mutation, donner naissance à des molécules possédant des caractères néfastes pour un hôte particulier (nécrose de la tomate).

De plus, il est généralement admis que des séquences étrangères, ou les reliquats de constructions génétiques, placés aux extrêmités des molécules d'ARN viral peuvent affecter la réplication dudit ARN (EP 242 016). Par conséquent, les nécessaires étapes de coupure, ligation et purification lors des constructions génétiques réalisées par ces autéurs, compliquent et rallongent considérablement le processus de préparation des plantes transgéniques.

C'est pourquoi, la présente invention concerne un procédé simple et rapide permettant la création de plantes transgéniques, rendues tolérantes, de façon durable, aux phytovirus par l'intégration dans leur génome d'une information génétique codant pour l'expression de molécules d'acide nucléique satellite. La simplicité du procédé selon la présente .invention résulte notamment du fait que la séquence d'ADN codant pour l'expression de l'acide nucléique satellite est introduite dans le génome sous la forme d'une seule unité monomérique et que, en outre, cette unité monomérique possède, de préférence, à ses deux extrémités, des séquences étrangères ou résidus de séquences étrangères tels que les séquences oligo (dG), oligo (dC) ou oligo (dA), ou même des résidus de plasmides.

Ces séquences étrangères peuvent éventuellement aussi coder pour une protéine potentiellement intéressante dans le cadre de la présente invention.

Ainsi, la présente invention va à l'encontre des préjugés communément admis selon lesquels une unité monomérique ou la présence de molécules étrangères s'opposent à l'infectivité de molécules satellites.

Enfin, les plantes transgéniques obtenues par le procédé selon la présente invention résistent aux infections propagées, non seulement de façon artificielle par l'infection de VMC mais aussi aux infection propagées par la voie naturelle des pucerons.

De préférence, selon l'invention, les molécules satellites exprimées, sont des molécules d'ARN satellite.

Cet ARN satellite est particulièrement intéressant car il réduit beaucoup la synthèse du génome viral dans la plante infectée (KAPER et TOUSIGNANT, 1977 ; MOSSOP et FRANCKI, 1979 ; TAKANAMI, 1981 ; JACQUEMONT et LEROUX, 1982).

Pour tous les hôtes testés, sauf pour la tomate, cette réduction de la réplication virale est corrolaire avec une atténuation importante des symptômes induits par le virus tout seul. La réponse biologique de la tomate rend possible de distinguer au moins deux groupes d'ARN satellite en fonction du fait que la plante développe des symptômes atténués - ce qui est valable pour d'autres hôtes - ou la nécrose létale (JACQUEMOND et LOT, 1981 ; KAPER et al., 1981). L'étude de l'interférence entre ces deux types d'ARN satellite a montré que les plantes de la tomate infectées avec une souche contenant un ARN satellite non nécrogène sont protégés contre l'infection avec une souche contenant un satellite nécrogène (JACQUEMOND, 1982).

L'information génétique codant pour l'expression de cet ARN satellite est, de préférence, introduite dans le génome des plantes, sous la forme d'une molécule de cADN transcrit reverse de l'ARN satellite.

Ainsi donc, on sait qu'il existe au moins deux groupes d'ARN satellites pouvant provoquer chez la tomate soit une atténuation des symptômes de filiformisme induits par le VMC (ARN " non nécrotique" (R)-), soit le développement de nécrose létale (JACQUEMOND et al., 1981 ; KAPER et al., 1981 ; PALUKAITIS et al., 1984) (ARN "nécrotique" (I 17 N)). Indépendamment de leurs caractéristiques biologiques (nécrotiques et non nécrotiques), les formes monomériques des cADN présentent les mêmes activités biologiques et peuvent être utilisées pour les mêmes fins par la présente invention.

Les ARN satellites appartenant à ces deux groupes ont été séquencés (RICHARDS et al., 1978 ; COLLMER et al., 1983 ; GORDON et al., 1983 ; AVILA - RINCON et al., 1986 ; GARCIA ARENAL et al.

3

1987) et partagent un important degré d'homologie excepté pour un type japonais qui diffère dans sa région centrale (HIDAKA et al., 1984).

Aucune relation simple ne pouvant être établie entre la séquence nucléotidique des ARN satellites et leur fonction biologique, on a cloné et séquencé les ADN complémentaires satellites des deux ARN satellites isolés. Or, il s'est avéré que ces ADN complémentaires satellites sont infectieux, c'est-à-dire, qu'ils produisent après co-inoculation avec un génome helper, un ARN capable de réplication et possédant les propriétés biologiques de l'ARN satellite natif.

L'efficacité de l'ADN complémentaire dépend :
- de sa forme dans l'inoculum (plasmide circulaire ou linéaire ou ADN complémentaire excisé),
- de la forme du virus helper (ARN viral ou virions).

De préférence selon l'invention, le virus utilisé est le virus de la mosaïque du concombre (VMC) mais de nombreux virus peuvent être utilisés à condition qu'un ARN ou ADN satellite puissent leur être associés.

Les vecteurs utilisés par les constructions génétiques nécessaires au procédé de la présente invention peuvent être quelconques. De préférence, on utilise Agrobacterium rhizogenes ou Agrobacterium tumefaciens. De même, comme on le verra dans la partie exemplifiée ci-après, les techniques utiles aux préparations des vecteurs sont connues de l'homme de l'art, et se caractérisent par leur simplicité.

La présente invention est exemplifiée ci-après à l'aide des figures suivantes :

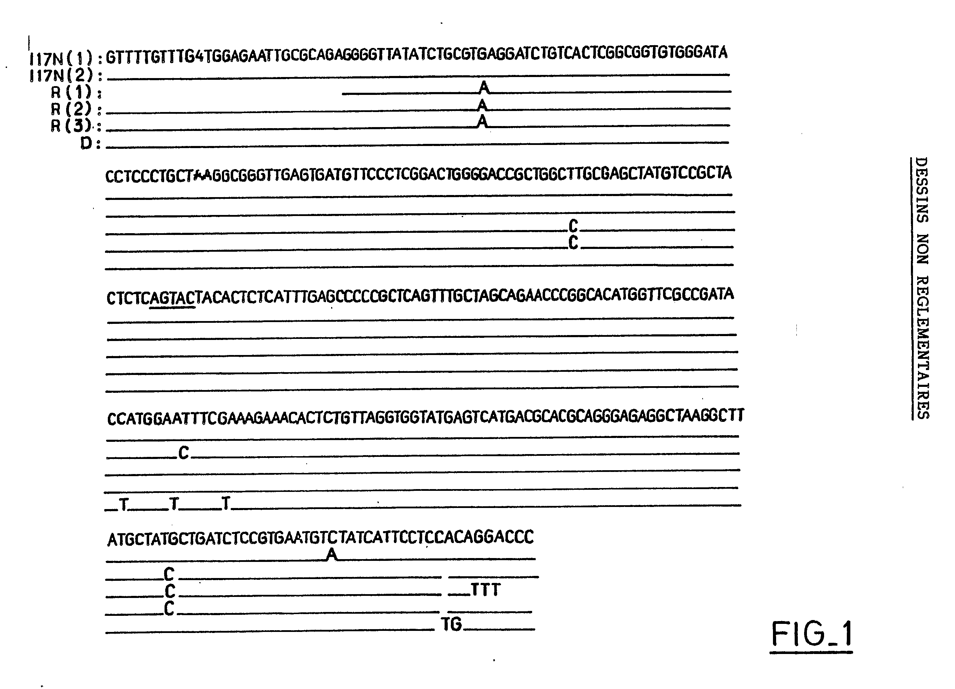

La figure 1 représente la comparaison des séquences de cADNS satellites I17 N, R et D.

4 copies de cADN de longueur totale de l'ARN satellite I 17 N ont été séquencées ; parmi elles, trois sont représentées par la séquence I 17 N (1) et l'une par la séquence I 17 N (2). R (1) et (2) correspondent à deux copies différentes de l'ARN satellite R. R (1) est une copie partielle s'allongeant à partir du résidu 28 jusque l'extrémité 3´. R (3) est un clone recombinant construit à partir des clones (1) et (2) digéré par Sca I (la position de ce site à l'intérieur de la séquence est soulignée) et porte l'extrémité 3´ du cADN du clone (1) et l'extrémité 5´ du cADN du clone (2). La séquence D, déduite de la séquence connue d'ARN (RICHARDS et al. 1978) est utilisée à titre de référence. Une ligne horizontale signifie aucun changement alors qu'une ligne interrompue indique une deletion. Les substitutions de base sont représentées par les lettres correspondantes.

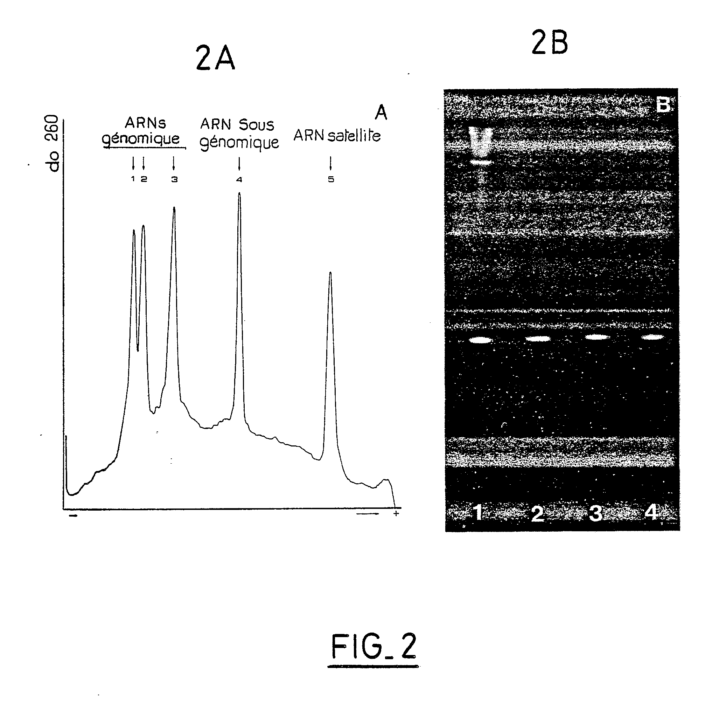

La figure 2 représente l'analyse de l'ARN produit après infection avec un inoculum comportant un cADN I 17 N.

2A correspond à l'électrophorégramme de l'ARN extrait à partir des nucléoprotéines récupérées et analysées dans un gel de polyacrylamide à 2,4 %.

2B correspond à l'analyse d'un ARN satellite par chromatographie sur oligo (dT) cellulose. Les différentes fractions d'ARN ont subi une électrophorèse sur un gel de polyacrylamide à 5 % dans l'urée 8 M.

Ligne 1 : ARN viral total.
Ligne 2 : 5ème composant purifié sur un gradient de saccharose.
Ligne 3 : fraction non retenue sur oligo (dT) cellulose.
Ligne 4 : ARN satellite natif.

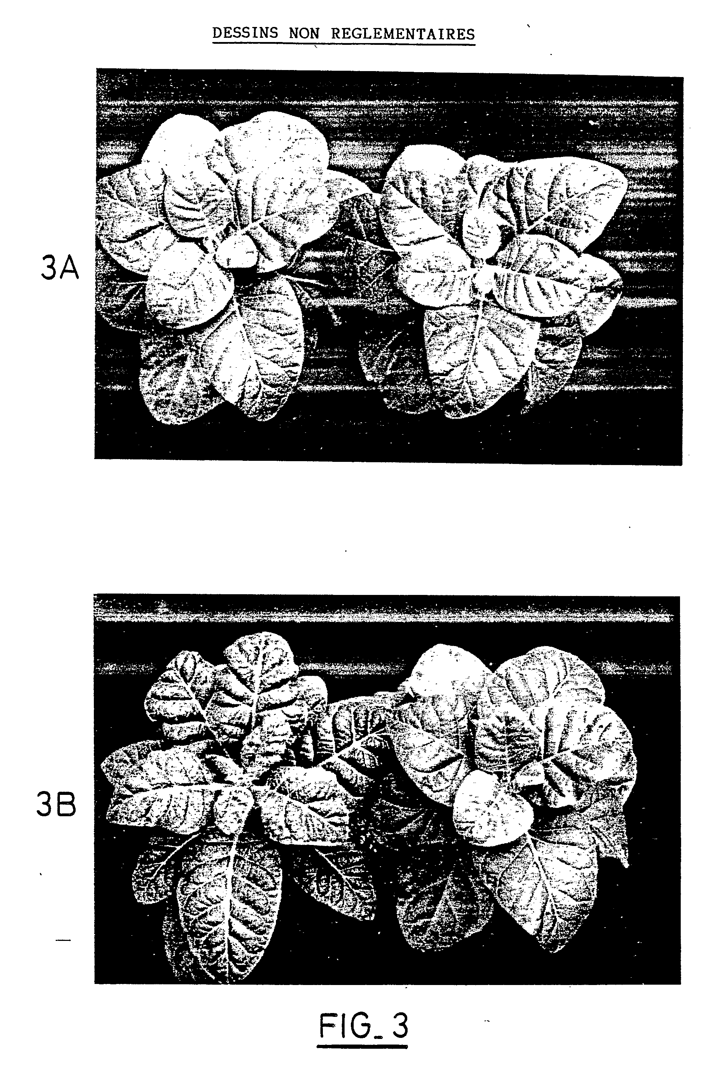

La figure 3 représente chez le tabac, les effets de l'expression du gène de l'ARN satellite sur le développement des symptômes du VMC.

Les tabacs témoins sont représentés sur la figure 3B. Les tabacs avec cADN satellite sont représentés sur la figure 3A.

Sur ces deux figures, la feuille de gauche correspond à la plante inoculée, par le VMC indemne de satellite (souche I17F), la feuille de droite correspond à la plante témoin non inoculée.

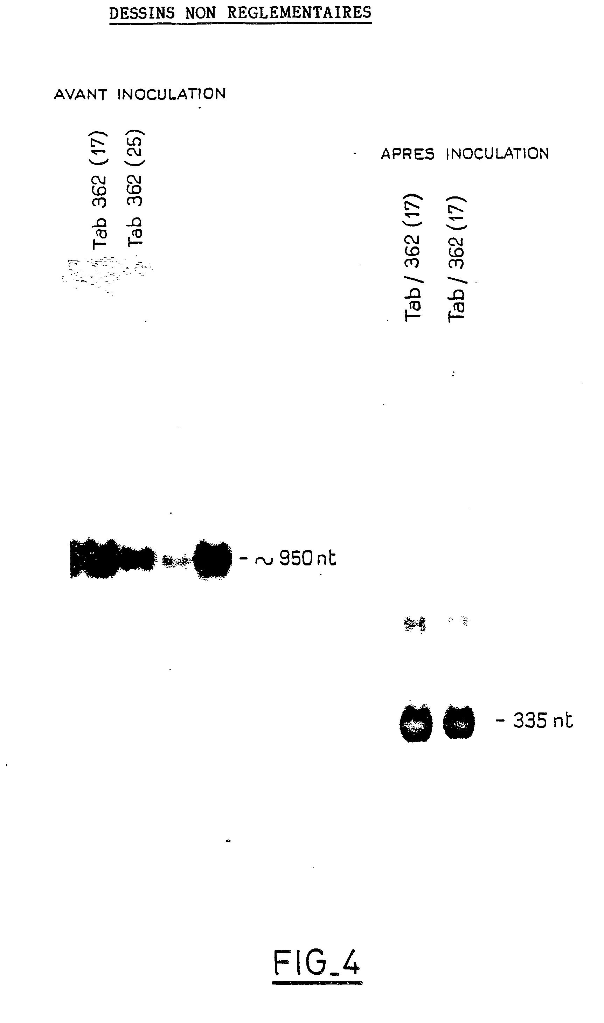

La figure 4 représente l'expression du précurseur de l'ARN satellite dans les tabac/362 et sa maturation après inoculation par le VMC.

Sur cette figure, les électrophorèses réalisées montrent que :
- avant l'inoculation du VMC, le transcrit, correspondant au précurseur de l'ARN satellite, possède environ 950 nucléotides (soit approximativement 1 kb),
- après l'inoculation du VMC, le transcrit a subi une maturation et correspond à l'ARN satellite, il possède environ 335 nucléotides.

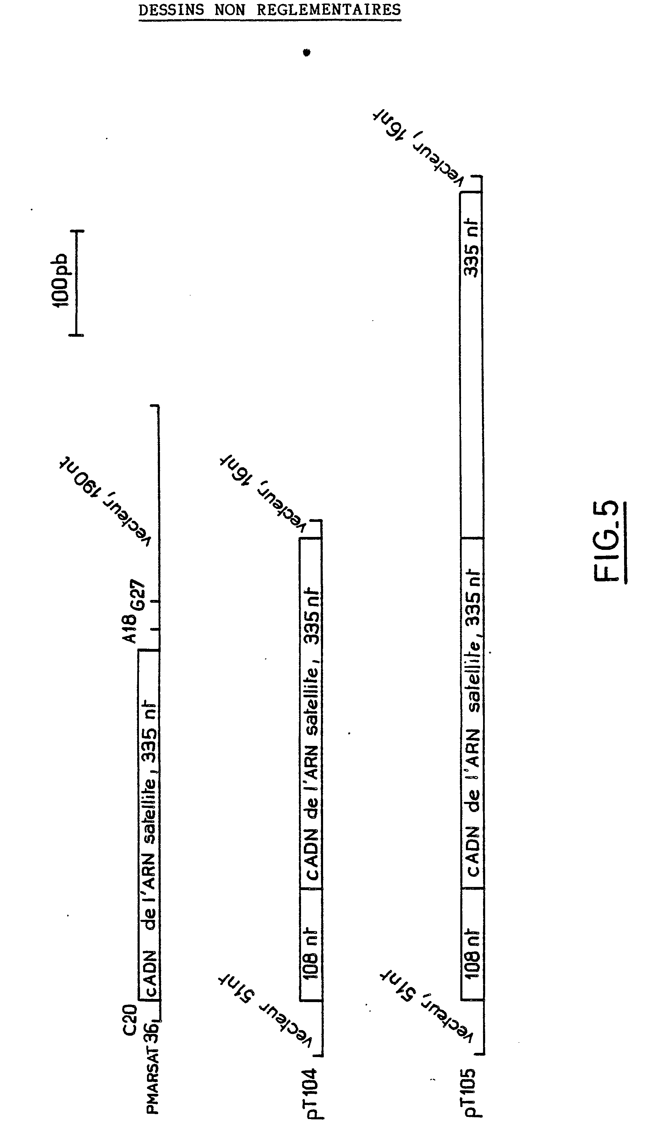

La figure 5 représente la structure de l'ADN inséré dans les vecteurs d'expression pour créer des gènes codant pour des précurseurs d'ARN satellites. On compare la structure du pMARSAT36 avec les vecteurs utilisés par BAULCOMBE et al. (1986) et HARRISON et al. (1987) soit pT104 et pT 105.

Sur cette figure "nt" correspond à "nucléotides". Les 108 nt des vecteurs pT 104 et pT 105 proviennent de l'extrémité 3´ de l'ARN satellite. Le vecteur pMARSAT 36 est notamment caractérisé par les queues homopolymériques suivantes : 20 résidus C ($C_{20}$), 18 résidus A ($A_{18}$) et 27 résidus G ($G_{27}$) et un fragment de 190 nucléotides dérivés du vecteur utilisé pour le clonage du cADN pAT 153.

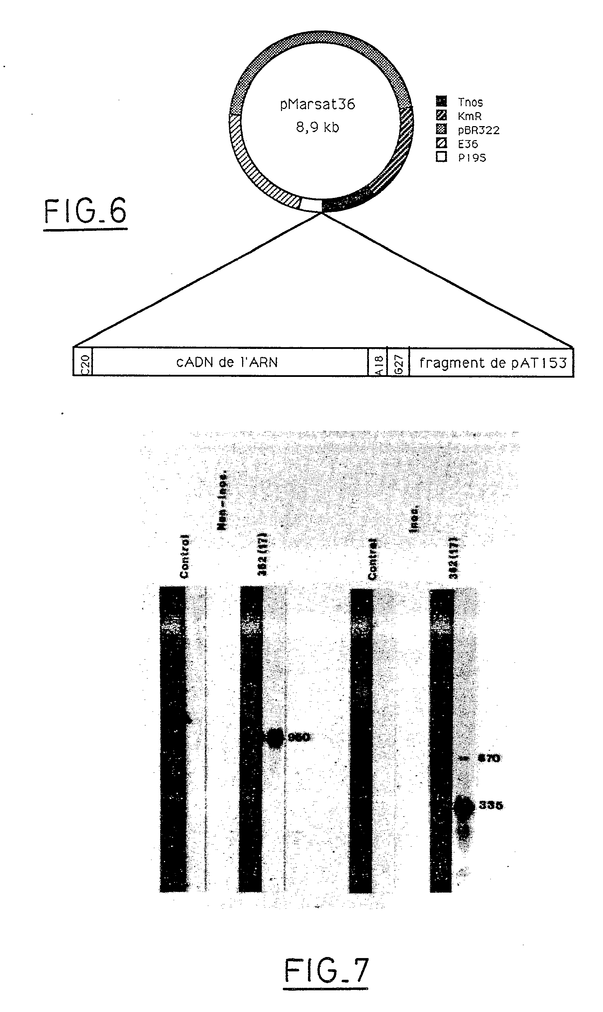

La figure 6 représente la carte du pMARSAT 36, vecteur intermédiaire portant le gène de l'ARN satellite : Le fragment BamH1 de 0,59 kb portant une simple copie du cADN de l'isolat I17N de l'ARN satellite est inséré au site de clonage unique BamH1 du vecteur d'expression pMarcel19. Ce dernier porte le fragment 36 de EcoR1 de la région TL du pRiA4, ainsi que les séquences pBR322, permettant la recombinaison avec soit pRIA4, ou avec certains plasmides Ti désarmés tels que pGV3850. Les queues oligo(dA), oligo(dC) et oligo (dG), et le fragment de pAT153 sont le résultat de l'amorce par le cADN et du clonage dans pAT153 (JACQUEMOND et LAUQUIN, 1988). Tnos = région 3' de la nopaline synthétase, KmR = gène de résistance à la kanamycine à partir de Tn5,E36 = fragment 36 de EcoR1 de la région TI du pRIA4, P18S = promoteur caMV 19S.

La figure 7 représente la maturation du transcript à partir de pMARSAT36 dans les plantes transgéniques Tob/362 (17) et l'amplification de l'ARN satellite résultant après infection par le VMC :
Les ARN totaux sont extraits à partir des feuilles des plantes soit non inoculées (à gauche) ou inoculées par le VMC (à droite). Les plants contrôles sont transformés avec le plasmid Ri de type sauvage. Les lignes à partir du gel coloré de bromure d'éthidium unique et le blot de Northern correspondant, en utilisant le cADN de l'ARN satellite à titre de preuve, sont montrées par paires. 15 microgrammes d'ARN sont appliquées par ligne. L'autoradiogramme de l'ARN à partir des plantes non-inoculées et des plantes inoculées par le VMC contrôle est exposé pendant 48 heures à -80° avec des rayons intenses alors que,celui après inoculation, est exposé pendant 20 minutes à température ambiante sans rayons. La taille de l'ARN, indiquée en nucléotides, est estimée par interpolation à partir de la migration des ARN marqueurs standars (Echelle d'ARN de Boehringer).

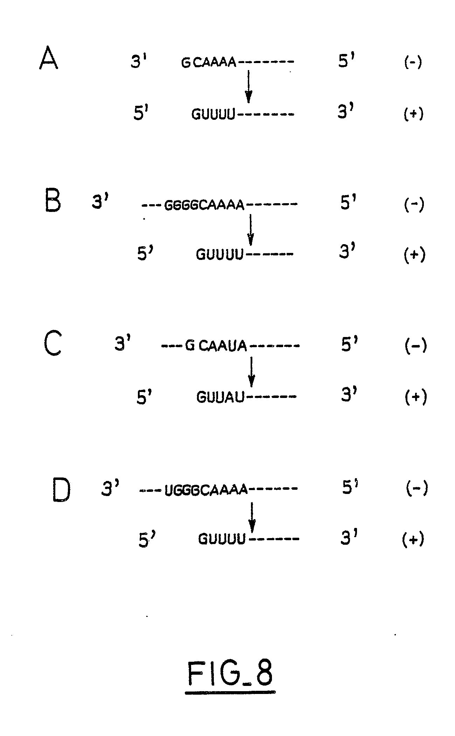

La figure 8 représente une comparaison des sites présumés d'initiation de synthèse du brin d'ARN (+) sur l'ARN satellite et génomique du VMC (-) :
A) Dans les formes réplicatives de l'ARN satellite du VMC, l'extrémité 3' du brin (-) a un G non apparié ; le brin correspondant (+) est présumé s'initier au C adjacent (COLLMER et KAPER, 1985).
B) La queue oligo (dG) 3' du transcript du précurseur de l'ARN satellite (-) recrée une paire GC au site de l'initiation interne présumée du brin d'ARN satellite (+).
C) Le site de l'initiation interne présumée pour la synthèse du brin sous génomique (+) est à la paire GC sur le brin (-) de l'ARN-3 (COLLMER et KAPER, 1985).
D) Les transcripts du précurseur de l'ARN satellite oligomérique dans les plantes transformées de BAULCOMBE et al. (1986) recréent aussi une paire GC au site d'initiation du brin (+).

L'exemple suivant montre notamment qu'une protection est obtenue après l'introduction dans les plants hôtes en utilisant un vecteur basé sur le plasmide Ri, d'un gène chimérique contenant une copie monomérique d'ARN satellite du VMC.

La présence de séquences non virales correspondant aux queues homopolymériques ajoutées pour la synthèse et le clonage de l'ADNc ainsi qu'à un fragment du vecteur de clonage (JACQUEMOND et LAUQUIN, 1988) n'interfère pas avec l'activité biologique des transcripts. De plus, ces plants sont également tolérants lorsqu'on les infecte avec des pucerons virulatns, les vecteurs naturels du VMC.

## EXEMPLE

## A - MATERIEL ET METHODES

### 1 - Souches de virus

La souche VMC I 17 F ne possède pas d'ARN satellite et induit chez la tomate de graves symptômes de filiformisme. La souche VMC I 17 N contient un ARN satellite "nécrotique" chez la tomate, alors que la souche VMC R contient un satellite "non nécrotique" chez la tomate. Les purifications des virions, ARN viraux, ARN satellites et électrophorèse sur les gels de polyacrylamide à 2,4 % ont été décrits antérieurement (JACQUEMOND et al., 1981).

### 2 - Plasmide et souches bactériennes

Les digestions par l'endonucléase de restriction, ligations, transformation de E. coli, les maxi et mini-préparations d'ADN plasmidique et autres techniques de biologie moléculaire usuelles sont décrites chez MANIATIS et al. (1982).

3 - L'ARN satellite de la souche de VMC I 17 N a été isolé, puis polyadénylé à son extrémité 3'. Puis le cADN correspondant a été synthétisé.

La polyadénylation d'ARN satellite a été effectuée dans un volume réactionnel de 100 $\mu$l contenant 50 mM de Tris-Hcl (pH 7,9), 10 mM de MgCl$_2$, 250 mM de NaCl, 2,5 mM de MnCl$_2$, 0,25 mM d'ATP, 40 unités de RNasin, 45 pmoles d'ARN, 1,5 unités de E. coli poly (A) polymérase pendant 10 min à 37°C. La synthèse du premier brin d'ADN a été réalisée selon la méthode de GUBLER et HOFFMAN (1983). La synthèse du second brin d'ADN complémentaire a été réalisée par la méthode de OKAYAMA and BERG (1982) avec seulement des légères modifications. Le mélange réactionnel de 50 $\mu$l comportait 20 mM de Tris-HCl (pH 7,5), 5 mM de MgCl$_2$, 10 mM d'acétate de NH$_4$, 100 mM de KCl, 40 mM de chaque dNTP, l'ensemble de la préparation ARN-ADN précédente, 9 unités de RNase H/ml, 240 unités d'E. coli ADN polymérase 1/ml et a été incubé successivement à 12°C et 22°C pendant 1 heure chaque fois.

Après chaque étape, la réaction a été stoppée par addition d'EDTA (concentration finale 20 mM) et les acides nucléiques ont été extraits avec du phénol, deux fois avec du phénol-chloroforme et précipités dans de l'acétate de NH$_4$ 2M avec de l'éthanol.

4 - Clonage : Des queues oligo (dG) ont été ajoutées au cADN et des queues oligo (dG) au vecteur pAT 153 au site EcoRV, permettant le clonage du cADN

Le vecteur pAT 153 a été totalement coupé avec EcoRV. L'ADN a été extrait par du phénol et sédimenté à travers un gradient de saccharose dans le but d'assurer une très petite quantité d'ADN plasmidique non coupé dans la préparation d'ADN recombinant (NORGARD et al., 1980). L'addition d'une queue homopolymérique dCTP au plasmide a été effectuée dans un volume réactionnel de 100 $\mu$l comportant 28 mM d'acide cacodylique - 120 mM de base Tris ajusté à pH 7,6 avec KOH, 5 mg/ml de BSA 0,1 mM de dCTP, 60 unités de déoxynucléotidyl transférase terminale/ml pendant 10 minutes à 22°C. La réaction a été stoppée par addition d'EDTA (concentration finale 5 mM). Une moyenne de 20 nucléotides a été ajoutée dans ces conditions. L'addition d'une queue homopolymérique dGTP à l'ADN complémentaire a été réalisée de manière similaire, en présence de 1 mM de dGTP et 540 unités d'enzyme/ml pendans 1 heure à 37°C. Le vecteur et l'ADN complémentaire ont été hybridés sans purification supplémentaire sous les conditions de GUBLER et HOFFMAN (1983). Les cellules compétentes d'E. Coli souche RR1 ont été préparées et transformées par la procédure de HANAHAN (1983). Les clones recombinants ont été choisis pour la résistance à l'ampicilline et la sensibilité à la tétracycline. L'insertion de l'ADN complémentaire dans le site EcoRV de pAT 153 par liaison homopolymérique G:C a généré un nouveau site BamH1 à cette position (GUBLER et al. 1983). La digestion du plasmide recombinant avec BamH1 a généré un fragment qui contenait l'ADN complémentaire plus 190 pb à partir du plasmide d'ADN et qui était long d'au moins 600 pb si les ADN complémentaires étaient une copie complète de l'ARN.

5 - Séquençage du cADN

La séquence de l'ADN complémentaire déduite des séquences d'ARN publiées montre un site Scal unique et commun en position 151. Les deux fragments contenant l'ADN complémentaire obtenus par digestion du plasmide recombinant avec BamH1 et Scal ont été clonés dans la forme réplicative de M 13 tg 131 (KIENY et al., 1983) coupée par BamH1/EcoRV et séquencés par la méthode de terminaison de chaîne de Sanger et al. (1977)

6 - Tests biologiques : Pour démontrer l'activité biologique du cADN par co-infection de plantes sensibles avec du virus sans satellite

Dix plants de tomates jeunes, Lycopersicon esculentum v. Monalbo, (âgés de 14-16 jours) ont été inoculés mécaniquement avec un mélange comportant soit 40 $\mu$g/ml d'ARN viral purifié (ARN-1-4) soit 100

μg/ml de virions de la souche VMC 17F (cette souche libre d'ARN satellite est maintenue chez la tomate dans le but de la conserver libre d'ARN satellite ou de détecter l'ARN satellite s'il s'accumule pendant la réplication virale), et de l'ADN. La nature de l'ADN (plasmide non coupé ou linéarisé, ADN complémentaire excisé) et sa concentration ont été spécifiées pour chaque expérience. La présence d'un ARN satellite nécrotique a été révélée par le développement de la nécrose chez la tomate. Celle de l'ARN non nécrotique est basée sur la capacité de cet ARN satellite à protéger la plante contre une infection ultérieure par un isolat inducteur de nécrose (JACQUEMOND et al., 1982). Les dix plants ont été surinfectés avec une préparation d'ARN total I 17 N (40 μg/ml) deux semaines après la première inoculation. Les résultats sont exprimés en pourcentages de plants nécrosés ( % PN) ou plants protégés (% PP), respectivement.

## 7 - Création de plantes transgéniques exprimant un gène codant pour un monomère d'ARN satellite :

Le fragment BamH1 comportant le cADN additionné des 190 pb du plasmide a été cloné dans le vecteur d'expression pMarcel19 décrit dans ROBAGLIA et al. (1987).

Le plasmide résultant pMarsat36, a été introduit dans l'ADN-T du plasmide Ri de la souche A4RS d'Agrobactérium rhizogenes par conjugaison triparentale pour créer la souche d'A. Rhizogenes, Marsat 362 (voir figures 5 et 6 pour pMarsat 36).

La souche Marsat362 a été utilisée pour inoculer des segments de tige de tabac (Nicotiana tabacum xanthi nc, souche XHFD8) ; parmi les racines transformées induites et mises en culture, trois clones ont été étudiés plus en détail, Tab/362 (17), Tab/362 (18), Tab/362 (25).

Les techniques utilisées pour transférer le vecteur intermédiaire pMarsat36 chez A. rhizogenes A4, pour inoculer les segments de tige de tabac et pour régénérer les plants transformés à partir des racines transformées résultantes sont décrites dans TEPFER et CASSE-DELBART (1987). Brièvement, le pMarsat36 est introduit dans la souche A. rhizogenes A4 par accouplement triparentaux en utilisant le pRK2013 comme plasmide Helper. La souche A. rhizogènes résultante portant le co-intégrat entre pMarsat36 et pRiA4 est utilisée pour inoculer in vitro les segments de tige de tabac stérilisés en surface. Les racines transformées sont décontaminées, maintenues comme clones d'organes et induites pour régénérer des petites plantes.

## 8 - Isolation de l'ARN total et analyse "Northern"

L'ARN total est isolé à partir des cultures de racine axénique et des plants de tabac transformés avant et après inoculation, en utilisant la technique de CHIRGWIN et al. (1979), suivie par un gradient au CsCl selon GLISIN et al. (1974). Des échantillons d'ARN sont analysés par électrophorèse dans des gels de dénaturation au formaldéhyde (LEHRACH et al. (1977)) et transférés aux membranes de nylon de AMERSTRAM HYBOND-N selon le protocole du fabricant. Les témoins marqués au phosphore[32] sont préparés à partir des fragments isolés de cADN de l'ARN satellite en utilisant un amorçage oligonucléotidique randomnisé comme décrit par FEINBERG et VOGELSTEIN (1983).

Les ARN totaux des trois cones de racines transformées par Marsat 362 sont extraits. L'analyse par les techniques classiques d'hybridation moléculaire (technique "Northern") a mis en évidence un transcrit d'approximativement 1 kb correspondant au précurseur de l'ARN satellite (voir figure 4).

## 9 - Etude du comportement des tabacs transformés vis à vis du VMC

Des plants de tabac transgéniques sont régénérés à partir de 3 clones de racines transformées exprimant le gène de l'ARN satellite, puis multipliés in vitro pour obtenir un nombre suffisant de plantules génétiquement identiques pour les tests suivants

La réponse à l'infection par le VMC libre de satellite (souche I17F) de ces plants des trois clones Tab/362 a été comparée à celle de deux types de témoins (tabacs non transformés, tabacs transformés par la souche sauvage d'A. rhizogenes à savoir tabac portant le Ri-T-DNA sans gène d'ARN satellite) selon plusieurs critères :

a) taux de réplication du satellite,

b) taux de réplication du virus,

c) expression de symptômes de maladie après infection par une souche de VMC indemne de satellite.

## 10 - Transmission par les pucerons

Cette transmission est réalisée comme décrit par LECOQ et al. (1979) avec Myzus persicae ou Aphis gossypii. La source virale est du melon (cucumis melo variété Cantaloup charentais) infecté 7 jours avant par une souche I17F. Au moins 10 pucerons virulents sont déposés sur chaque plant de tabac dans le but d'assurer l'infection. Deux heures après, les pucerons sont tués par fumigation et les plants sont conservés dans une serre exempte d'insectes.

## 11 - Détermination de la propagation virale dans les plants de tabac expériment

Le virus est purifié selon LOT et al. (1972) 7 jours après l'inoculation. L'ARN est extrait à partir des nucléoprotéines par la méthode au phénol -SDS et analysé sur un gel de polyacrylamide à 2,4 %. Les extraits bruts des plants infectés sont préparés dans un tampon phosphate (4 ml pour 1 g de feuille). Une partie est testée pour les propriétés biologiques sur des plants de tomate jeunes. Le développement de nécrose par ces plants est caractéristique de la présence d'un ARN satellite nécrogène dans l'inoculum. Le restant de l'extrait est conservé à 20°C pour la quantification de la protéine capsidique.

La quantité de protéine capsidique est quantifiée par la méthode Elisa sous la forme du double sandwich d'anti-corps (Clark et Adams, 1977) en utilisant des réactifs préparés contre la souche LQ du VMC, qui appartient au même groupe que I17F (don de H.LOT). Des extraits bruts sont ensuite dilués 1 000 à 16 000 fois selon qu'ils contiennent ou non de l'ARN satellite.

Des contrôles sont basés sur les virions I17F purifiés dilués à 1-200 µg/ml dans un extrait brut de tabac non infecté ; des échantillons contrôles sont ensuite dilués comme les expérimentaux.

## B - RESULTATS

## 1 - Séquences des cADN satellites

Les séquences des cADN satellites I17N et R sont présentées sur la figure 1 en comparaison avec la séquence connue de l'isolat D (RICHARD et al. 1978) à titre de référence. En fonction des clones, 5 ou 6 substitutions ont été observées entre les cADN des deux ARN "nécrotiques" (I17N et D) (positions 218, 224, 229, 326, 327 et 314). Parmi ces changements, trois ont été aussi notés dans la séquence de l'ARN R "non nécrotique" (positions 218, 229, et 327). De plus, cet ARN présentait des différences supplémentaires. Trois substitutions (positions 44, 126 ou 225 pour les clones 2 et 1 respectivement, 295) et une délétion d'une base (position 326). Alors que la copie partielle de cet ARN possède les mêmes 7 derniers résidus à l'extrémité 3' que le ADN de I 17 N, la copie totale présente une extrémité 3' modifiée : Ces 7 résidus sont absents et 3T se trouvent aux positions 329 à 331. Dans le but de déterminer si ces modifications ont présenté une variation de l'ARN satellite R ou une mauvaise initiation de la transcription reverse, on a construit un recombinant entre les deux copies du cADN. Le nouveau clone (3) contenait un cADN ayant la terminaison 5' de la copie de longueur totale précédente et la terminaison 3' de la copie de longueur partielle. Finalement, ce cADN différait du cADN de I17N (1) par 3 substitutions (positions 44, 126 et 297) et par la délétion d'une base (position 326).

## 2) Construction d'un gène d'ARN satellite

Un gène dont le transcript correspond à un précurseur monomérique de l'ARN satellite du VMC a été construit, en utilisant le vecteur d'expression intermédiaire pMarcel19 (ROBAGLIA et al., 1987) qui possède un site de clonage BamH1 unique entre le promoteur 19S du virus de la mosaique Cauliflower (CaMV) et un fragment portant les signaux de polyadénylation du gène de la Nopaline synthétase. La famille pMarcel de vecteurs peut être insérée par co-intégration soit, dans un plasmide RiA4 d'une souche Agrobactérium Rhizogenes type sauvage, soit dans certains plasmides désarmés Ti tels que pGV3850 (Zambryski et al., 1983).

Le clone d'ADNc utilisé correspond à une simple copie de l'isolat nécrogène de 335 nucléotides de l'ARN satellite L17N, cloné et séquencé par JACQUEMOND et LAUQUIN (1988). Le fragment BamH1 de ce clone avec la structure suivante (oligo dC/satellite/ oligo dA/oligo dG/fragment de vecteur) a été cloné dans le vecteur pMarcel19 d'expression de la plante intermédiaire pour créer pMarsat36 (figure 6). On a utilisé le système du plasmid Ri décrit en détail par TEPFER et CASSE-DELBART (1987) pour introduire le pMarsat36 dans les plantes de tabac. Les transformants portant ce gène sont appelés Tob/362 (n), où (n) est le numéro de clone.

### 3) Expression du gène de l'ARN satellite dans les plantes avant et après l'infection par le VMC

Plusieurs clones de racines de tabac transformés génétiquement portant le gène de l'ARN satellite sont régénérés et les petits plants résultants sont propagés in vitro. Les blots "Northern" d'ARN total de ces plants (figure 7), lorsqu'on les essaye avec le cADN de l'ARN satellite, montrent un transcript unique de taille attendue, approximativement de 0,95 kb (insert de 0,59 kb + 3' non codant et poly A de 0,35 kb). Le niveau d'expression est variable selon les clones ; par exemple, les clones Tob/362 (17) et Tob/362 (25) ont des niveaux de transcript bien supérieurs aux clones Tob/362 (18).

Comme on peut le voir sur la figure 7, une bande visible dans le bromure d'éthidium apparaît à 0,33 kb dans l'ARN extrait à partir des plantes Tob/362 après inoculation avec le virus du VMC. La densitométrie de plusieurs gels d'ARN de différentes plantes Tob/362 infectées par le VMC montre que cette bande représente essentiellement 10 - 12 % de l'ARN de la plante totale. Les blots northern de ces gels essayés avec le cADN de l'ARN satellite montrent que la nouvelle bande correspond à l'ARN satellite ; l'ARN satellite a été correctement traité à partir du transcript du précurseur pour donner l'ARN satellite monomérique d'une seule longueur qui est grandement amplifié par le virus. Des formes dimériques de 0,67 kb sont aussi visibles. Le niveau d'ARN satellite n'est pas corrélé avec le niveau de transcript du précurseur ; Autant d'ARN satellite est présent chez les plantes Tob/362 (18) infectées que chez les plantes Tob/362 (17) et Tob/362 (25).

### 4 - Propriétés biologiques des cADN satellites

L'ARN satellite possède une activité biologique très élevée. Il est répliqué dans des conditions optimales lorsqu'il est présent à une concentration de 4,5 pM dans l'inoculum et provoque encore le développement de nécrose (ou symptômes de filiformisme atténués) chez certaines des plantes infectées lorsqu'il est présent à des doses inférieures (JACQUEMOND et al.,1982). Pour la validité des tests biologiques il était nécessaire d'être sûr que les symptômes modifiés des tomates infectées ne résultaient pas d'une contamination des préparations d'ARN viraux ou de virions par un ARN satellite. Dans chaque expérience concernant un cADN d'un ARN "nécrotique", 10 plants ont été inoculés avec l'une de ces préparations seule et 10 autres avec le même inoculum comportant 5 à 20 μg/ml d'une préparation purifiée de pAT 153. Aucun de ces contrôles n'a développé les symptômes caractéristiques de la présence d'un ARN satellite de type quelconque. Pour l'étude des copies de cADN de l'ARN "non nécrotique", une troisième série de plants contrôles a été d'abord infectée avec la souche I17F ,puis surinfectée avec la souche inductrice de nécrose. Toutes les plantes sont mortes de la nécrose, ce qui confirme que aucun ARN satellite "non nécrotique" n'a contaminé les préparations virales et ceci indique que la surinfection a été réalisée avec succès.

La co-inoculation du virus Helper et d'une copie de cADN de longueur totale de l'ARN satellite I17N peut induire la nécrose parmi les plantes infectées. Les résultats de plusieurs tests sont résumés dans le tableau 1.

TABLEAU 1

| Propriétés biologiques du cADN de l'ARN satellite I 17 N | | | | | | |
|---|---|---|---|---|---|---|
| | Virus Helper | | | | | |
| ADN plasmidique µg/ml | ARN 40µg/ml | | | Virions 100 µg/ml | | |
| | Nb.P[a] | %IP[a] | %NP[a] | Nb.P[a] | %IP[a] | %NP[a] |
| 100 | - | - | - | 70 | 100 | 57,1 |
| 50 | 20 | 0 | - | 80 | 100 | 61,2 |
| 20 | 20 | 0 | - | 80 | 100 | 77,5 |
| 10 | 30 | 86,7 | 15,4 | 80 | 100 | 67,5 |
| 5 | 52 | 100 | 17,3 | 60 | 100 | 48,3 |

a : Nb.P : Nombre de plantes inoculées ; %IP : Pourcentage de plantes infectées ; % NP : Pourcentage de nécrose parmi les plantes infectées.

Les données obtenues avec les inoculums contenant l'une des 2 copies de longueur totale ne différaient pas significativement et ont été cumulées.

L'une des caractéristiques inhabituelles des préparations d'ADN, à des concentrations supérieures à 10 µg/ml, était leur capacité d'inhiber l'infection par les ARN viraux. D'autre part, lorsque l'ADNC était à des concentrations inférieures, peu de plantes seulement ont développé le symptôme caractéristique de la réplication et l'expression de l'ARN nécrotique. Ce probléme a été surmonté par l'utilisation de virions purifiés à la place d'ARN viraux.

Dans ces conditions, l'infection de toutes les plantes a été assurée quelque soit la quantité d'ADN et l'activité biologique de l'ADN a été améliorée. Cependant, le cADN est apparu 8 000 fois moins actif que l'ARN satellite lui-même si l'on considére qu'un pourcentage similaire de plantes nécrosées a été obtenu par l'inoculation d'une solution comportant l'ARN satellite à 0,45 pM (JACQUEMOND et al., 1982) ou 10 µg/ml de plasmide recombinant (approximativement à 3,8 nM de cADN). La réduction des proportions de plantes nécrosées lorsque l'ADN était présent à des concentrations supérieures à 20 µg/ml a constitué un résultat inattendu qu'il reste à expliquer dans tous les cas, il ne semble pas possible d'induire la nécrose de toutes les plantes dans les conditions rapportées ici.

L'orientation de l'insert du cADN dans le vecteur PAT 153 n'a pas joué un rôle pour son expression (tableau 2).

TABLEAU 2  Effet de l'orientation de l'insert du cADN et de la linéarisation du plasmide recombinant sur les propriétés biologiques du cADN de l'ARN satellite I 17 N

| cADN | Orientation de l'insert[a] | | | |
| | I | | II | |
| | Nb.P[b] | %NP[b] | Nb.P[b] | %NP[b] |
|---|---|---|---|---|
| plasmide non coupé 20 µg/ml | 120 | 68,3 | 30 | 53,3 |
| plasmide coupé EcoRI 20 µg/ml | 100 | 7,0 | 30 | 56,7 |
| plasmide coupé SphI 20 µg/ml | 60 | 66,7 | -c | - |
| plasmide coupé SalI 20 µg/ml | 100 | 55,0 | 30 | 13,3 |
| fragment BamHI 2 µg/ml | 60 | 10,0 | - | - |

Le virus a été apporté sous la forme de virions purifiés (100 µg/ml).

a :

E : EcorI, B : BamHI, Sp :SphI,
S : SalI.
La flèche indique l'orientation de la copie d'ADN dont la transcription donnerait un brin d'ARN(+).

b: Nb.P : Nombre de plantes inoculées, % NP : Pourcentage de plantes nécrosées.

c: - : non testé.

Des résultats similaires ont été obtenus avec le cADN du STNV (Virus satellite de la nécrose du tabac) (VAN EMMELO et al.,1987) et de viroides (CROSS et al., 1983 ; TABLER et al., 1984). Ainsi, aucune séquence particulière du vecteur plasmidique ne semblait être impliquée dans le procédé d'infection de ces cADN . Lorsque le cADN a été apporté comme plasmide recombinant linéarisé, le site de coupure était important. Les résultats présentés dans le tableau 2 montrent que la linéarisation devait arriver près de l'extremité 3' du brin (+) du cADN pour garder le cADN actif. Comme l'ARN satellite se réplique probablement en utilisant la voie métabolique ARN-ARN, son cADN doit servir in vivo comme matrice pour une ARN-polymérase ADN-dépendante de l'hôte. En outre, les résultats suggèrent que l'ARN transcrit doit être un brin (+). Le fragment contenant le cADN généré par digestion totale avec BamHI s'est révélé moins actif que le plasmide non coupé (tableau 2). Bien que l'efficacité du précédent pourrait être augmentée en

11

réduisant la concentration des virions (50 μg/ml) ou en le co-inoculant avec des ARN viraux, pas plus de 20 % des plantes ont développé des nécroses.

Les tests de l'activité biologique du clone 2 du cADN satellite R étaient tous négatifs. Aucun ARN possédant l'une des deux fonctions de l'ARN satellite ne pouvait être récupéré à partir des tomates inoculées. Toutes ces plantes ont développé des symptômes de filiformisme sévères. Ceci suggerait que l'extrêmité 3' modifiée de cette copie de cADN est due probablement à une mauvaise copie de transcriptase reverse. De plus, l'inoculation des plants de tomate avec un inoculum comportant de cADN clone 3 a permis la protection complète de certains d'eux contre une infection ultérieure par la souche inductrice de nécrose. Cette possibilité est caractéristique de la présence d'un ARN satellite "non nécrotique" dans la plante protégé (JACQUEMOND et al., 1982). Comme montré dans le tableau 3, le cADN de l'ARN satellite R à présenté une activité biologiques comparable à celle des copies de longueur totale d'ARN satellite I17N. En particulier, un maximum d'efficacité a été observé lorsque le plasmide a été introduit à 20 μg/ml dans l'inoculum.

TABLEAU 3

| Propriétés biologiques du cADN de l'ARN R : protection contre un surinfection avec le VMC | | |
|---|---|---|
| ADN plasmidique μg/ml | Nb.P[a] | %PP[b] |
| 100 | 50 | 28 |
| 50 | 50 | 38 |
| 20 | 80 | 55 |
| 10 | 50 | 42 |
| 5 | 50 | 34 |

a : Nb.P : Nombre de plantes inoculées
b : %PP : Pourcentage de plantes protégées après surinfection avec la souche I17 N.

Le virus a été apporté sous la forme de virions purifiés. (100 μg/ml). Pendant chaque expérience, pour contrôler l'expression du cADN, 10 plantes ont été infectées avec un inoculum contenant 20 μg/ml de cADN I 17 F : une moyenne de 53,3 % des plantes ont développé des nécroses.

Les nucléoprotéines purifiées à partir des plants de tomate qui ont développé de la nécrose après infection avec un inoculum contenant du cADN I17N comprenaient un 5ème composant migrant comme l'ARN satellite pendant l'électrophorèse à travers un gel de polyacrylamide de 2,4 % et représentant 18 % de l'ARN encapsidé (figure 2A). Cet ARN a été ensuite purifié par centrifugation sur gradient de saccharose et testé par sa capacité de liaison à l'oligo (dT) cellulose, il ne portait pas la queue poly (A) (longue de 18 résidus) présente à l'extrêmité 3' du brin ( + ) du cADN utilisé dans ce cas. Par conséquent, la queue poly (G) située en 3' du poly (A) serait aussi absente. De plus, après une électrophorése à travers un gel de polyacrylamide à 5 % dans des conditions dénaturantes qui permettraient d'observer nettement une différence de 20 à 45 bases (longueur de chaque queue ), l'ARN possède la même mobilité électrophorétique que l'ARN satellite natif (figure 2B). En outre, il a induit le même syndrôme de nécrose typique sur les tomates que l'ARN satellite natif. L'analyse de la descendance virale a aussi été effectuée à partir de tomates inoculées avec un inoculum comportant le cADN R. Dans une première expérience, 10 plantes ont été analysées pour la présence d'ARN satellite à l'intérieur des nucléoprotéines alors que 10 autres ont été surinfectées avec l'inoculum nécrotique. Un 5ème composant migrant comme l'ARN satellite sous des conditions dénaturantes a été obobservé dans 8 préparations parmi 10 dans le premier cas alors que 7 plantes parmi 10 ont été protégées contre l'infection dans le dernier cas. Dans une expérience indépendante, on a observé que toutes les plantes protégées ont montré la présence d'un ARN type ARN satellite à l'intérieur des virions extraits.

5 - Comportement des plantes transgéniques vis à vis du VMC (inoculation mécanique)

Le comportement des deux types de plants contrôles vis à vis du VMC (figure 3) est comme suit : Les feuilles inoculées et les premières feuilles non inoculées développent des symptômes faibles (éclaircissement des veines et mosaique diffuse) pendant la première semaine après l'infection. Puis, les feuilles suivantes montrent une mosaique sévère ; la surface des feuilles est aussi réduite. Les symptômes développés par les deux feuilles suivantes sont légèrement plus doux et ceux développés par les suivantes sont encore très sévères. Les plants transformés exprimant le gène de l'ARN satèllite montrent des symptômes faibles similaires aux contrôles pendant la semaine suivant l'infection. Après cette semaine, les symptômes développés par les feuilles supérieures sont extrêmement atténués ; souvent les plants ne montrent pratiquement aucun symptôme (figure 3). Une protection similaire est observée pour les transformants des trois clones.

La réplication virale comme on le juge par les champs de nucléoprotéines virales purifiées sept jours après l'infection et la quantification sérologique de la protéine capsidique est décrite dans le tableau 5. La réplication virale est grandement réduite chez les plantes exprimant le gène de l'ARN satellite, puisque 3 à 4 fois moins de virus peuvent être récupérés à partir de ces plantes et la quantité de capside est approximativement 10 % de celle isolée à partir des deux types de plants contrôle. Cette diminution est corrélée avec une accumulation importante d'ARN satellite dans les nucléoprotéines virales (plus de 50 % de l'ARN encapsidé), alors qu'aucun ARN satellite n'est détecté dans les préparations de virions récupérées à partir des plants de contrôle infectés. Les mêmes résultats sont obtenus pour les trois clones de transformants exprimant le gène d'ARN satellite. Ces résultats confirment ceux obtenus par l'analyse Northern de l'ARN total de la plante montrée ci-dessus (figure 7). Les propriétés biologiques de cet ARN sont déterminées en inoculant des plants de tomates jeunes avec un extrait brut à partir de plants de tabac infectés. Tous les plants infectés avec un inoculum préparé à partir de transformants exprimant le gène de l'ARN satellite ont montré une nécrose léthale, alors que ceux infectés avec un inoculum préparé à partir de plants contrôles ont développé des symptômes de feuilles atténuées sévères caractéristiques de l'absence d'ARN satellite dans cet inoculum.

a) Lors d'une infection par le VMC des Tab/362, le transcrit précurseur d'un kb est mature pour donner un ARN satellite de taille (335 b) et d'activité biologioque normale, qui est très fortement répliqué(voir figure 4).

b) Le taux de réplication du virus lui-même, par contre, est fortement inhibé (réduit à 20 % du niveau des témoins).

c) La gravité des symptômes est atténuée par rapport aux témoins. (figure 3).

d) La protection conférée semble durable dans le temps. Il y a une diminution de la gravité de l'infection; il ne s'agit pas seulement d'un retard dans le développement de la maladie.

Les résultats sont représentés dans le tableau 4 suivant.

TABLEAU 4

|  | Virus [a]<br>mg/ g feuille | Capside virale [b]<br>µg/g tissu |
|---|---|---|
| Tabacs transformés avec le cADN satellite | ⌈ 687<br>⎮ 566<br>⌊ 614 | 101 |
| Tabacs transformés sans cADN satellite | 1 900 | 880 |
| Tabacs non transformés | 2 330 | 754 |

a) Rendement évalué après purification des nucléo-protéines virales 7 jours après l'infection. L'analyse en gel de polyacrylamide de ces préparations montre que, dans le cas de plantes exprimant le cADN satellite, au moins 50 % de l'ARN viral encapsidé est de l'ARN satellite.

b) Valeurs estimées par dosage immunoenzymatique de la protéine capsidique (ELISA).

6 - Inoculation par les pucerons

Les transformants à partir des clones Tob/362 (17) et Tob/362 (25) aussi bien que les trois types de plants contrôles (non transformé, transformé avec le plasmide de type Ri sauvage, transformé avec le plasmide de type Ri portant un gène chimérique sans rapport avec les virus) sont sauvés et les plants de première génération ont été testés pour leur susceptiblité à être infectés par les pucerons, le vecteur naturel du VMC. Dans la progéniture, les transformants peuvent être distingués par leur phénotype modifié. La modification est relativement douce chez les Tob/362(17) et relativement prononcé chez les plants contrôles A4(4), 314(1) et Tob/362(25). Dans le but de voir si la modification peut interférer avec la transmission virale, les plants contrôles tout seuls sont analysés dans une première série d'expérience. Les résultats sont présentés tableau 6, expérience 1 et montrent que les deux types de plants contrôles partagent une réponse similaire à l'infection par le VMC quel que soit leur phénotype. Dans les expériences suivantes, aussi bien les contrôles que les plantes Tob/362 (17) que les plantes Tob/362 (25) sont testées. Comme on le voit dans le tableau 6, expérience 2, tous les types variés de plants contrôles montrent des symptômes de mosaïque sévères et ne produisent pas· d'ARN satellite comme on le juge par les symptômes développés par les plants de tomate inoculés à partir de ces plants. D'un autre côté, tous les plants portant le gène de l'ARN satellite, développent seulement des symptômes atténués et produisent de l'ARN satellite nécrogène. Comme on peut le voir avec des plants inoculés mécaniquement, la réplication du virus ainsi que mesuré par la quantification de capside est aussi considérablement réduite chez les plants inoculés par des pucerons si on le compare aux plants non transformés.

Tableau 5.

| Comportement vis à vis du V.M.C. de plantes exprimant le gène de l'ARN satellite du V.M.C. | | | | | |
|---|---|---|---|---|---|
| | Plantes contrôles | | Plantes exprimant le gène de l'ARN | | |
| | T[a] | NT[a] | 362(17) | 362(18) | 362(25) |
| Nombre de plantes | 4 | 13 | 12 | 2 | 4 |
| Rendement moyen de virus(b) | | | | | |
| (mg virus/kg feuille) | 1887 | 2329 | 687 | 566 | 614 |
| Quantification de capside(c) | | | | | |
| 7 jours après l'inoculation | 760 | 755 | 108 | 97 | 77 |
| 14 jours après l'inoculation | n.a. | 775 | 53 | 45 | 60 |

(a) T = plantes contrôles transformées, NT = plantes contrôles non transformées
(b) Rendement viral calculé chez Lot et al. (1972)
(c) Concentration capsidique, mesurée sérologiquement, exprimée en $\mu$g de protéine capsidique par g de tissu de feuille
n.a. = non analysé

Tableau 6.

| Résultats de l'infection par les pucerons de la lignée à partir de plantes transformées ou non avec le gène de l'ARN satellite | | | | | | | | | |
|---|---|---|---|---|---|---|---|---|---|
| | Expérience 1 | | | Expérience 2 | | | | | |
| Génotype des plantes parent (a) | Xanthl/Xanthl | 314(1)/Xanthl | | Xanthl/Xanthl | A4(4)/A4(4) | 362(17)/Xanthl | | 362(25)/Xanthl | |
| Phénotype de la lignée avant inoculation (b) | N | N | T' | N | T' | N | T | N | T' |
| Nombre de plantes testées | 21 | 17 | 5 | 11 | 16 | 14 | 4 | 6 | 11 |
| Quantification capsidique (c) | 548 | 681 | 620 | 790 | 790 | 325 | 40 | 275 | 18 |
| Gravité des symptomes (d) | + | + | + | + | + | + | - | + | - |
| % nécrose parmi les plants de tomates inoculés avec l'extrait brut du tabac infecté | 0 | 0 | 0 | 0 | 0 | 0 | 100 | 0 | 100 |

(a) Xanthi = contrôles non transformés ; 314(1)/Xanthi = plantes hemizygotes transformées avec un gène de contrôle; A4(4)/A(4) = plantes transformées par pRi homozygotes

(b) N = phénotype de type sauvage ; T = phénotype léger pRi-transformé ; T' = phénotype pRi-transformé sévère,

(c) µg de capside par g de tissude feuille, 2 semaines après l'infection,

(d) + = mosaïque sévère ; - = pas de mosaïque ou mosaïque très atténuée ;

(e) 2 ou 3 plants de tomates sont infectés dans les 1ère et 2ème expériences respectivement, avec un extrait brut de plants de tomate.

## REFERENCES

- AVILA-RINCON, M.J., COLLMER, C.W. and KAPER, J.M. (1986) virology, 152,446-454 ;
- BAULCOMBE, D.C., SAUNDERS, G.R., BEVAN, M.W., MAYO M.A. and HARRISON, B.D. (1986) Nature 321, 446-449 ;
- BAUSCH,J.N., KRAMER, F.R., MIELE, E.A., DOBKIN, C. and MILLS, D.R. (1983) J. Biol. Chem. 258, 1978-1984 ;
- ALQUHIST, P., FRENCH, R., JANDA, M. and LOESCH-FRIES, L.S. (1984) Proc. Natl. Acad. Sci. USA, 81, 7066-7070 ;
- BEVAN, M.W., FLAVELL, R.B. and CHILTON, M.D. (1983) Nature, 304, 184-187 ;
- BRUENING G., BUZAYAN, J., GERLACH, W. and HAMPEL,A. (1988) In von Wettstein, D. and Chua, N.H. (ed.), Plant Molecular Biology. NATO ASI Series A : Life Sciences Vol 140., pp. 495-502 ;
- COLLMER, C.W., TOUSIGNANT, M.E. and KAPER, J.M. (1983) Virology, 127, 230-234 ;

- COLLMER, C.W. and KAPER. J.M. (1985), Virology, 145, 249-259

- COLLMER, C.W. and KAPER, J.M. (1986) Biochem. Biophys. Res. Commun., 135, 290-296 ;

- CLARK, M.F. and ADAMS, A.N. (1977) J. gen. Virology, 34, 475-483 ;

- CHIRGWIN, J.M., PRZYBYLA, A.E., MACDONALD, R.J. and RUTTER, W.J. (1979) Biochemistry, 18, (5294-5299) ;

- COMAI, L., FACIOTTI, D., HIATT, W.R., THOMPSON,G., ROSE, R.E. and STALKER, D.M. (1985) Nature, 317, 741-744 ;

- CRESS, D.E., KIEFFER, M.C. and OWENS, R.A. (1983) Nucleic Acids Res. 11, 6821-6835 ;

- CUOZZO, M., O'CONNELL, K.M. KANIEWSKI, W., FANG, R.X., CHUA, N.H. and TUMER, N.E. (1988) Bio/Technology, 6, 549-557 ;

- DE BLOCK, M. BOTTERMANN, J., VANDEWIELE, M., DOCKX,J., THOEN, C., GOSSELE, V., RAO MOVVA, N., THOMPSON, C., VAN MONTAGU, M. and LEEMANS,J. (1987) EMBO J.,6, 2513-2518.

- DIAZ-RUIZ.J.R. and KAPER, J.M. (1977) Virology, 80, 204-213 ;

- DIENER, T.O. (1986) Proc. Natl. Acad. Sci. USA, 83, 58-62;

- DOUINE, L., QUIOT, J.B.,.MARCHOUX, G; and ARCHANGE, P. (1979) Ann. de phytopathol. 11, 439-475 ;

- FEINBERG, A.P. and VOGELSTEIN, B. (1983) Anal. Biochem. 132, 6-13 ;

- FISCHHOFF, D.A., BOWDISH, K.S., PERLAK, F.J., MARRONE, P.G., McCORMICK, S.M., NIEDERMERYER, J.G., DEAN, D.A., KUSANO-KRETZMER, K., MAYER, E.J. ROCHESTER, D.E., ROGERS, S.G. and FRALEY, R.T. (1987) BIO/TECHNOLOGY, 5, 807-813.

- FRALEY, R., ROGERS, S., HORSCH, R., SANDERS, R., FLICK, J. ADAMS,S., BITTNER, M., BRAND, L., FINCK, C., FRY,J., GALLUPPI, G., GOLDBERG, S., HOFFMANN, N. and WOO, S.(1983) Proc. Natl. Acad. Sci. USA, 80, 4803-4807 ;

- GERLACH, W.L., BUZAYAN, J.M., SCHNEIDER, I.R. and BRUENING, G. (1986) Virology, 151, 172-185 ;

- GERLACH, W.L., LLEWELLYN,D. and HASELOFF, J. (1987) Nature, 328,802-805 ;

- GLISIN, V., CRKVENJAKOV. R. and BYUS, C. (1974) Biochemistry 13, 2633-2637 ;

- GORDON, K.H.J. and SYMONS, R.H. (1983) Nucleic Acids Res., 11, 947-960 ;

- GOULD, A.R. PALUKAITIS, P., SYMONS, R.H. and MOSSOP, D.W. 1978) Virology, 84, 443-445 ;

- HANAHAN, D. (1983) J. Mol. Biol., 166, 557-580 ;

- HARRISON, B.D., MAYO, M.A. and BAULCOMBE, D.C. (1987) Nature 328, 799-802 ;

- HEMENWAY, C., FANG, R.X. KANIEWSKI, W.K., CHUA, N.H., TUMER, 1988) EMBO J.7, 1273-1280 ;

- HERRERA-ESTRELLA, L., DEPICKER,A., VAN MONTAGU, M. and SCHELL J. (1983) Nature, 303, 209-213 ;

- HILDER, V.A., GATEHOUSE, A.M.R., SHEERMAN, S.E., BARKER, R.F. and BOULTER,D. (1987) Nature, 300, 160-163 .

- JACQUEMOND, M. and LOT, H. (1981) Agronomie, 1, 927-932 ;

- GARCIA-ARENAL, F., ZAITLIN, M. and PALUKAITIS, P. (1987) Virology, 158, 339-347 ;

- HIDAKA, S., ISHIHAWA, K., TAKANAMI, Y., KUBO, S. and MIURA, K. (1984) FEBS Lett., 174, 38-42 ;

- GUBLER, U; and HOFFMAN, B.J. (1983) Gene, 25, 263-269 ;

- JACQUEMOND,M. (1982) C.R. Acad. Sci. Paris, III, 294, 991-994 ;

- JACQUEMOND, M. and LEROUX, J.P. (1982) Agronomie, 2, 55-62;

- JACQUEMOND, M. and LAUQUIN, G.J.M. (1988) Biochem. Biophys. Res. Comm., 151, 388-395 ;

- JAYNES, J.M., XANTHOPOULOS, K.G., DESTEFANO-BELTRAN, L. and DODS, J.H. (1987) BioEssays 6, 263-270 ;

- JEFFERSON, R.A. KAVANAGH, T.A. and BEVAN, M.W. (1987) EMBO J.6. 3907-3909 ;

- KAPER, J.P., TOUSIGNANT, M.E. and LOT, H. (1976) Biochem. Biophys. Res. Comm., 72, 1237-1243 ;

- KAPER, J.M. and TOUSIGNANT, M.E. (1977), Virology, 80, 186-195 ;

- KAPER, J.M., TOUSIGNANT, M.E. and THOMPSON, S.M. (1981), Virology, 114, 526-533 ;

- KIENY, M.P., LATHE, R. and LECOCQ, J.P. (1983) Gene, 26, 91-99 ;

- LECOQ, H., COHEN, S., PITRAT, M. and LABONNE, G. (1979, Phytopathology, 69, 1223-1225 ;

- LEHRACH, H., DIAMOND, WOZNEY, J.M. and BOEDTKER, H. (1977) Biochemistry, 16, 4743-4751 ;

- LOECH-FRIES, L.S., MERLO, D., ZINNEN, T., BURHOP, L., HILL, K., KRAHN,K., JARVIS, N., NELSON, S and HALK, E. (1987) EMBO J.,6, 1845-1851 ;

- LOT, H., MARROU, J., QUIOT, J.B. and ESVAN, C. (1972) Ann. Phytopathol., 4, 25-38 ;

- LOT, H., RICHARdS, K. and JONARD, G. (1977) FEBS Lett., 80, 395-399 .

- MANIATIS, T., FRITSCH, E.F. and SAMBROOK, J., Eds. (1982) Molecular Cloning : a Laboratory Manual. Cold Spring Harbor Laboratory, Cold Spring Harbor, NY ;

- MILLER, W.A., DREHER, T.W. and HALL, T.C. (1985), Nature, 313, 68-70 ;

- MILLER W.A., BUJARSKI, J.J., DREHER, T.W. and HALL, T.C. (1986) J. Mol. Biol., 187, 537-546 ;

- MOSSOP, D.W. and FRANCKI. R.I.B. (1979), Virology, 95, 395-404 ;
- NORGARD, M.V., TOCCI, M.J. and MONAHAN, J.J. (1980) J. Biol. Chem., 255, 7665-7672 ;
- PALUKAITIS, P. and ZAITLIN, M. (1984) Virology, 132, 426-435;
- RICHARDS, K. JONARD, G. JACQUEMOND, M. and LOT, H. (1978) Virology, 89, 395-408 ;
- OKAYAMA, H. and BERG, P. (1982) Mol. Cell. Biol., 2, 161-170 ;
- OMATA, T., KOHARA, M., SAKAI, Y., KAMEDA, A., IMURA, N. and NOMOTO, A. (1984) Gene, 32 1-10 ;
- POWELLABEL, P., NELSON, R.S., DE, B., HOFFMANN N., ROGERS S., FRALEY, R.T. and BEACHY, R.N. (1986) Science, 232, 738-743 ;
- SANGER, F., NICKLEN, S. and COULSON, A.R. (1977) Proc. Natl. Acad. Sci. USA, 74, 5463-5467 ;
- SHAH, D.M., HORSCH, R.B., KLEE, H.J. KISHORE, G.M., WINTER, J.A., TUMER, N.E.,
- HIRONAKA, C.M., SANDERS, P.R., GASSER, C.S., AYKENT,S., SIEGEL N.R., ROGERS, S.G. and FRALEY R.T. (1987) Science, 233, 478-481 ;
- TABLER, M. and SANGER, H.L. (1984) The EMBO Journal, 3, 3055-3062 ;
- TAKANAMI,Y. (1981) Virology, 109, 120-126 ;
- TEPFER, D. (1984) Cell, 37, 959-967 ;
- TEPFER, M. and CASSE-DELBART, F. (1987) Microbiological Sci. 4, 24-28 ;
- TUMER, N.E., O'CONNELL, K.M., NELSON, R.S., SANDERS, P.R., BEACHY, R.N., FRALEY, R ; T ; and SHAH, D.M. (1987) EMBO J.,6, 1181-1188 ;
- VAECK,M., REYNAERTS, A., HOFTE, H., JANSENS, S., DE BEUCKELEER, M., DEAN, C.;
- ZABEAU, M., VAN MONTAGU, M. and LEEMANS, J. (1987) Nature 328, 33-37 ;
- VAN EMMELO, J. AMELOOT, P. and FIERS, W. (1987) Virology, 157, 480-487 ;
- VAN DUN, C.M.P., BOL, J.F. and VAN VLOTEN-DOTING, L. (1987) Virology, 159, 299-305 ;
- WATERWORTH, H.E., KAPER, J.M. and TOUSIGNANT, M.E. (1979) Science, 204, 845-847 .
- ZAMBRYSKI, P., JOOS, H., GENETELLO, C., LEEMANS, J., VAN MONTAGU, M. and SCHELL, J. (1983) EMBO J.,2,2143-2150.
- ROBAGLIA, C., VILAINE, F., PAUTOT, V., RAYMOND, F., AMSELEM, J., JOUANIN, L., CASSE-DELBART, F. and TEPFER, M. (1987) Biochimie, 69, 231-237 ;
- ROBERTSON, H.D., ROSEN, D.L. and BRANCH, A.D. (1985) Virology 142, 441-447 .

**Revendications**

1. Procédé d'obtention de plantes transgéniques en vue de les rendre tolérantes de façon durable aux phytovirus par intégration dans leur génome d'une information génétique codant pour l'expression de molécules d'acide nucléique satellite, caractérisé en ce que la séquence d'ADN codant pour l'expression de l'acide nucléique satellite est introduite dans le génome sous la forme d'une seule unité monomérique dont les extrémités possèdent éventuellement des séquences étrangères.

2. Procédé selon la revendication 1, caractérisé en ce que l'acide nucléique satellite exprimé est une molécule d'ARN.

3. Procédé selon la revendication 2, caractérisé en ce que l'information génétique codant pour l'expression de l'ARN satellite est introduite dans le génome de la plante, sous la forme d'une molécule de cADN transcrit reverse de l'ARN satellite.

4. Procédé selon l'une des revendications 1 à 3, caractérisé en ce que les séquences étrangères sont des séquences oligo (dG), oligo (dC) ou oligo (dA).

5. Procédé selon l'une quelconque des revendications 1 à 3, caractérisé en ce que les séquences étrangères codent pour une protéine.

6. Procédé selon l'une quelconque des revendications 1 à 5, caractérisé en ce que le phytovirus est le virus de la mosaique du concombre (VMC).

7. Procédé selon la revendication 6, caractérisé en ce que l'unité monomérique introduite dans le génome est une molécule de cADN.

8. Procédé selon la revendication 7, caractérisé en ce que la molécule de cADN a été synthétisée à partir d'ARN polyadénylé puis doté de queues dG/dC à chacune de ses extrémités.

9. Procédé selon l'une des revendications 1 à 8, caractérisé en ce que l'information génétique est intégrée grâce à un vecteur de transformation

10. Séquence nucléotidique de virus non nécrogène, caractérisée en ce qu'elle correspond à une séquence R (1), R (2) ou R (3) selon la figure 1.

11. Vecteur de transformation utile au procédé selon la revendication 9, caractérisé en ce qu'il s'agit d'Agrobacterium rhizogenes ou d'Agrobacterium tumefaciens.

12. Plantes transgéniques obtenues par le procédé selon l'une quelconque des revendications 1 à 9.

```
I17N(1): GTTTTGTTTG4TGGAGAATTGCGCAGAGGGGTTATATCTGCGTGAGGATCTGTCACTCGGCGGTGTGGGATA
I17N(2): _____
   R(1): _____A_____
   R(2): _____A_____
   R(3): _____A_____
      D: _____

         CCTCCCTGCTAAGGCGGGTTGAGTGATGTTCCCTCGGACTGGGGACCGCTGGCTTGCGAGCTATGTCCGCTA
         _____
         _____C_____
         _____C_____
         _____

         CTCTCAGTACTACACTCTCATTTGAGCCCCCGCTCAGTTTGCTAGCAGAACCCGGCACATGGTTCGCCGATA
         _____
         _____
         _____
         _____

         CCATGGAATTTCGAAAGAAACACTCTGTTAGGTGGTATGAGTCATGACGCACGCAGGGAGAGGCTAAGGCTT
         _____
         _____C_____
         _____
         _T____T____T_____

         ATGCTATGCTGATCTCCGTGAATGTCTATCATTCCTCCACAGGACCC
         _____A_____
         _____C_____
         _____C_____ __TTT____
         _____C_____
         _____ TG_____
```

FIG_1

## 2A

## 2B

FIG_2

DESSINS NON REGLEMENTAIRES

3A

3B

FIG_3

AVANT INOCULATION

Tab 362 (17)

Tab 362 (25)

APRES INOCULATION

Tab / 362 (17)

Tab / 362 (17)

$- \sim 950\,nt$

$- 335\,nt$

# FIG_4

FIG.5

FIG.6

pMarsat36
8,9 kb

- Tnos
- KmR
- pBR322
- E36
- P195

| C20 | cADN de l'ARN | A18 | 627 | fragment de pAT153 |

FIG.7

FIG_8

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,Y | EP-A-0 242 016 (AGRICULTURAL GENETICS CO. LTD)<br>* En entier *<br>--- | 1-12 | C 12 N 15/00<br>A 01 H 1/00 |
| D,Y | CHEMICAL ABSTRACTS, vol. 106, 1987, page 181, résumé no. 170200q, Columbus, Ohio, US; J. VAN EMMELO et al.: "Expression in plants of the cloned satellite tobacco necrosis virus genome and of derived insertion mutants", & VIROLOGY 1987, 157(2), 480-7<br>* Résumé *<br>--- | 1-12 | |
| D,Y | CHEMICAL ABSTRACTS, vol. 97, 1982, page 416, résumé no. 124199u, Columbus, Ohio, US; M. JACQUEMOND: "Interference phenomena between the two types of satellite RNA of cucumber mosaic virus. Protection of tomato plants against lethal necrosis", & C.R. SEANCES ACAD. SCI., SER. 3 1982, 294(21), 991-4<br>* Résumé *<br>--- | 10 | |
| Y | CHEMICAL ABSTRACTS, vol. 104, 1986, page 150, résumé no. 142968m, Columbus, Ohio, US; C.W. COLLMER et al.: "Infectious RNA transcripts from cloned cDNAs of cucumber mosaic viral satellites", & BIOCHEM. BIOPHYS. RES. COMMUN. 1986, 135(1), 290-6<br>* Résumé *<br>---        -/- | 10 | **DOMAINES TECHNIQUES RECHERCHES (Int. Cl.4)**<br><br>C 12 N<br>A 01 H |

Le présent rapport a été établi pour toutes les revendications

| Lieu de la recherche | Date d'achèvement de la recherche | Examinateur |
|---|---|---|
| LA HAYE | 18-04-1989 | MADDOX A.D. |

Office européen
des brevets

RAPPORT DE RECHERCHE EUROPEENNE

Numero de la demande

EP 88 40 3181

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| O,P X | J. CELL. BIOCHEM. SUPPL. IOC, page 188, résumé no. L333; M. TEPFER et al.: "A chimeric gene coding for a cucumber mosaic virus (CMV) satellite RNA monomer confers tolerance to CMV infection" * Résumé * --- | 1-12 | |
| P,X | RAPPORT D'ACTIVITE INRA, 1987, pages 50-53; M. JACQUEMOND et al.: "Création de plantes transgéniques tolérantes au virus de la mosaique du concombre" * En entier * --- | 1-12 | |
| D,P X | BIOCHEMICAL AND BIOPHYSICAL RESEARCH COMMUNICATIONS, vol. 151, no. 1, 29 février 1988, pages 388-395, Academic Press Inc.; M. JACQUEMOND et al.: "The cDNA of cucumber mosaic virus-associated satellite RNA has in vivo biological properties" * En entier * --- | 10 | DOMAINES TECHNIQUES RECHERCHES (Int. Cl.4) |
| A | NATURE, vol. 321, 22 mai 1986, pages 446-449; D.C. BAULCOMBE et al.: "Expression of biologically active viral satellite RNA from the nuclear genome of transformed plants" * En entier * --- | 1-12 | |
| A | NATURE, vol. 328, 27 août 1987, pags 799-802; B.D. HARRISON et al.: "Virus resistance in transgenic plants that express cucumber mosaic virus satellite RNA" * En entier * --- -/- | 1-12 | |

Le présent rapport a été établi pour toutes les revendications

| Lieu de la recherche | Date d'achèvement de la recherche | Examinateur |
|---|---|---|
| LA HAYE | 18-04-1989 | MADDOX A.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

                

& : membre de la même famille, document correspondant

EPO FORM 1503 03.82 (P0402)

**Office européen des brevets**

# RAPPORT DE RECHERCHE EUROPEENNE

Numero de la demande

EP 88 40 3181

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | NATURE, vol. 328, 27 août 1987, pages 802-805; W.L. GERLACH et al.: "Construction of a plant disease resistance gene from the satellite RNA of tobacco ringspot virus" * En entier * --- | 1-12 | |
| A | CHEMICAL ABSTRACTS, vol. 105, 1986, page 392, résumé no. 111896c, Columbus, Ohio, US; X. YANG et al.: "Satellite RNA as a biological control agent of diseases caused by cucumber mosaic virus (CMV). III. Effect of satellite RNA on the content of double stranded viral RNA in CMV-infected tissues", & WEISHENGWU XUEBAO 1986, 26(2), 120-6 * Résumé * ----- | 1-12 | |

**DOMAINES TECHNIQUES RECHERCHES (Int. Cl.4)**

Le présent rapport a été établi pour toutes les revendications

| Lieu de la recherche | Date d'achèvement de la recherche | Examinateur |
|---|---|---|
| LA HAYE | 18-04-1989 | MADDOX A.D. |